(19) Europäisches Patentamt
European Patent Office
Office européen des brevets

(11) EP 2 110 119 A1

(12) EUROPEAN PATENT APPLICATION

(43) Date of publication:
**21.10.2009 Bulletin 2009/43**

(21) Application number: **08007489.1**

(22) Date of filing: **15.04.2008**

(51) Int Cl.:
***A61K 8/46*** *(2006.01)* ***A61K 8/44*** *(2006.01)*
***A61K 8/86*** *(2006.01)* ***A61Q 5/02*** *(2006.01)*
***A61Q 19/10*** *(2006.01)*

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **KPSS-Kao Professional Salon Services GmbH**
**64297 Darmstadt (DE)**

(72) Inventors:
- **Hoffmann, Martin**
  **64673 Zwingenberg (DE)**
- **Leukel-Schäfer, Diana**
  **64297 Darmstadt (DE)**

(74) Representative: **Grit, Mustafa**
**KPSS - Kao Professional Salon Services GmbH**
**Pfungstädterstraße 92-100**
**D-64297 Darmstadt (DE)**

(54) **Shear thickening cleaning composition**

(57) The present invention is related to an aqueous cleansing composition for keratin fibres especially human hair with shear thickening property. Specifically, present invention is on cleansing compositions for keratin fibres, especially for human hair, based on at least one anionic surfactant, especially anionic sulphate surfactant, at a concentration of 2 to 25% by weight, and further comprising at least one amino acid surfactant of the following structure

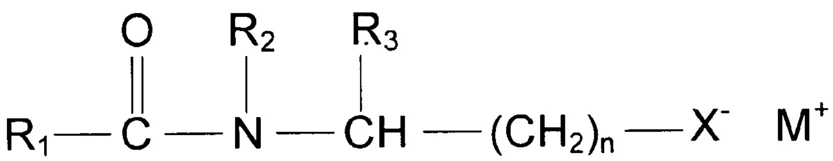

wherein $R_1$ is a saturated or unsaturated, straight or branched alkyl chain with 7 to 17 C atoms, $R_2$ is H or a methyl, $R_3$ is H, $COO^- M^+$, $CH_2COO^-$ M or COOH, n is 0 to 2, X is $COO^-$ or $SO_3^-$ and M is independent from each other H, sodium or potassium, at a concentration of 0.1 to 15%, by weight, and at least one ethoxylated monoglyceride of the following formula

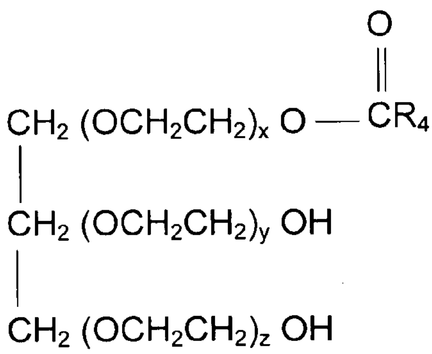

wherein $R_4$ is a saturated or unsaturated and branched or straight alkyl chain with a chain length of 7 to 21 C atoms and

EP 2 110 119 A1

x+y+z has a value of 50 to 200, at a concentration of at least 1% by weight, all values calculated to total composition.

## Description

The present invention is related to an aqueous cleansing composition for keratin fibres, especially human hair, with shear thickening property.

Cleansing compositions have been known for many years. Many patent applications and scientific publications deal with such compositions aiming at cleansing and especially improved conditioning effects. One of the requirements for a precise application of a cosmetic composition is that it should have certain consistency. Consistency of such composition must make optimal application at the site of usage possible with optimal dosage. Therefore, usually thickening agents are used for adjusting consistency of cosmetic compositions.

There are plenty of thickening agents available especially for cleansing compositions. Although the prior art developed quite extensively, it will make the formulators job easier in case a cleansing composition is thickened itself when pumping from a dispenser or pouring from a bottle or a tube under certain level of shear stress. In other words, it is a great advantage for developers of cleansing compositions, when a cleansing composition having its own consistency, but not the consistency required for optimal dosage and application, is thickened when taken out from a tube or a bottle by squeezing the tube or bottle strongly or when pumped out from a dispenser with relatively high shear stress.

Present inventors have surprisingly found that a cleansing composition comprising at least one anionic surfactant, at least one amino acid surfactant and at least one ethoxylated monoglyceride show excellent shear thickening property. This property is referred as rheopex behaviour in the rheology literature.

Accordingly, the first object of the present invention is a cleansing composition based on at least one anionic surfactant, especially anionic sulphate surfactant, at a concentration of 2 to 25% by weight, and further comprising at least one amino acid surfactant of the following structure

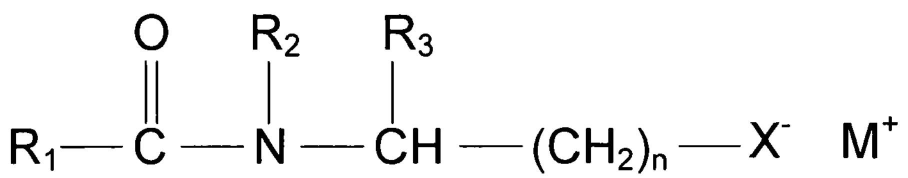

wherein $R_1$ is a saturated or unsaturated, straight or branched alkyl chain with 7 to 17 C atoms, $R_2$ is H or a methyl, $R_3$ is H, $COO^- M^+$, $CH_2COO^-$ M or COOH, n is 0 to 2, X is $COO^-$ or $SO_3^-$ and M is independent from each other H, sodium or potassium, at a concentration of 0.1 to 15%, by weight, and at least one ethoxylated monoglyceride of the following formula

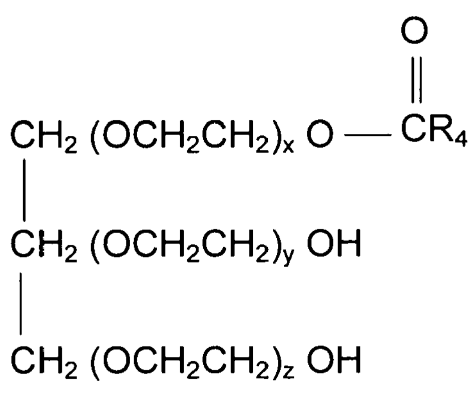

wherein $R_4$ is a saturated or unsaturated and branched or straight alkyl chain with a chain length of 7 to 21 C atoms and x+y+z has a value of 50 to 200, at a concentration of at least 1% by weight, all values calculated to total composition.

Within the meaning of the present invention, with the term anionic surfactant, anionic surfactants are meant other than those of amino acid surfactants.

With the term amino acid surfactants especially those surfactants are meant derived from taurate, glucamate,

alanin or alaninate, sarcosinate and aspartate.

Second object of the present invention is the use of at least one ethoxylated monoglyceride according to the above general formula at a concentration of at least 1% by weight, in a cleansing composition based on at least one anionic surfactant, especially sulphate type of anionic surfactant, at a concentration of 2 to 25% by weight, and further comprising at least one amino acid surfactant according to above general formula at a concentration of 0.1 to 15% by weight, all values calculated to total composition, as shear thickening agent.

With the term shear thickening, it is meant that composition has a lower consistency at zero or low shear stress than in presence of shear stress or higher shear stress values. In other words, composition has a lower consistency in an unmixed state than when it is mixed with for example spatula in a beaker.

Cleansing composition of the present invention comprises at least one ethoxylated monoglyceride according to the above general formula. In the preferred embodiment of the present invention $R_1$ is a saturated or unsaturated and branched or straight alkyl chain with a chain length of 11 to 17 C atoms, more preferably 13 to 17 C atoms and most preferably 15 to 17 C atoms and x+y+z has preferably a value of 60 to 200, more preferably 70 to 200 and most preferably 80 to 200.

Ethoxylated monogylcerides are known for their thickening ability in the area of hair cleansing compositions. For example WO 03/ 063818 A1 discloses ethoxylated glycerides as thickening agents in combination with ethoxylated fatty alcohol and ethoxylated partial gylcerides. WO 2004/024110 A1 and WO 03/013467 A1 disclose cleansing compositions comprising an ethoxylated monogylceride. However, all three patent publications do not mention shear thickening property of ethoxylated monoglycerides according to the above disclosed general formula in cleansing compositions.

Non-limiting suitable examples of ethoxylated monoglycerides are PEG-78 glyceryl cocoate, PEG-80 glyceryl cocoate, PEG-50 glyceryl isostearate, PEG-60 glyceryl isostearate, PEG-90 glyceryl isostearate, PEG-60 glyceryl stearate, PEG-120 glyceryl stearate, PEG-200 glyceryl stearate, PEG-80 glyceryl tallowate, PEG-82 glyceryl tallowate, PEG-130 glyceryl tallowate, PEG-200 glyceryl tallowate, and mixtures thereof. Preferred are PEG-78 glyceryl cocoate, PEG-80 glyceryl cocoate, PEG-60 glyceryl isostearate, PEG-90 glyceryl isostearate, PEG-60 glyceryl stearate, PEG-120 glyceryl stearate, PEG-200 glyceryl stearate, PEG-80 glyceryl tallowate, PEG-82 glyceryl tallowate, PEG-130 glyceryl tallowate and PEG-200 glyceryl tallowate. More preferred are PEG-78 glyceryl cocoate, PEG-80 glyceryl cocoate, PEG-90 glyceryl isostearate, PEG-120 glyceryl stearate, PEG-200 glyceryl stearate, PEG-80 glyceryl tallowate, PEG-82 glyceryl tallowate, PEG-130 glyceryl tallowate, PEG-200 glyceryl tallowate, and mixtures thereof. Most preferred are PEG-80 glyceryl cocoate, PEG-90 glyceryl isostearate, PEG-120 glyceryl stearate, PEG-200 glyceryl stearate, PEG-80 glyceryl tallowate, PEG-82 glyceryl tallowate, PEG-130 glyceryl tallowate, PEG-200 glyceryl tallowate, and mixtures thereof. The especially preferred ethoxylated monoglyceride is PEG-90 glyceryl isostearate which is available from Zschimmer & Schwarz under the trade name Oxetal VD 92.

Concentration of ethoxylated monoglyceride in the compositions of the present invention is preferably in the range of 1 to 15%, more preferably 1.5 to 12.5%, most preferably 1.5 to 7.5% and especially 2 to 5% by weight, calculated to total composition.

Cleansing composition of the present invention comprises at least one amino acid surfactant according to the general formula given above at a concentration of 0.1 to 15%, by weight, calculated to total composition. Preferably, the concentration of amino acid surfactant is from 0.25 to 10% by weight, more preferably 0.5 to 7.5% by weight and most preferably 1 to 5% by weight, calculated to total composition. In the preferred embodiment of the present invention $R_1$ in the general formula of amino acid surfactants disclosed above is a saturated or unsaturated, straight or branched alkyl chain with 9 to 17 C atoms, and more preferably 9 to 13 C atoms, $R_2$ is H or a methyl, $R_3$ is H, $COO^- M^+$, $CH_2COO^-$ M or COOH, n is 0 to 2, X is $COO^-$ or $SO_3^-$ and M is independent from each other H, sodium or potassium. It should be noted that alkyl chain includes also mixture of various alkyl groups as present especially in plant triglyceride derived alkyl chains such as cocoyl chain.

Suitably amino acid surfactant types are taurate, glutamate, alanin or alaninate, sarcosinate, aspartate surfactants, and mixtures thereof. Preferred are taurate, glutamate and sarcosinate surfactants and mixtures thereof. More preferred are taurates and glutamates and most preferred is glutamate type surfactants.

Suitable taurate surfactants are according to the general formula

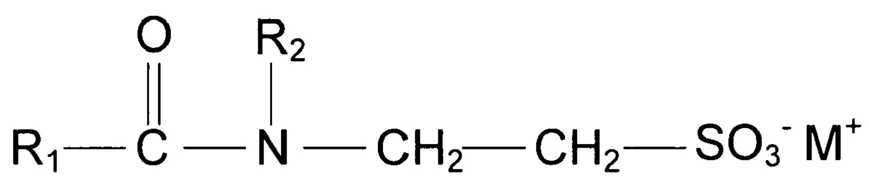

wherein $R_1$ is preferably a saturated or unsaturated, straight or branched alkyl chain with 7 to 17 C atoms, and more preferably 9 to 13 C atoms, $R_2$ is H or methyl, and M is H, sodium or potassium. Suitable examples are potassium cocoyl taurate, potassium methyl cocoyl taurate, sodium caproyl methyl taurate, sodium cocoyl taurate, sodium lauroyl taurate, sodium methyl cocoyl taurate, sodium methyl lauroyl taurate, sodium methyl myristoyl taurate, sodium methyl oleoyl taurate, sodium methyl palmitoyl taurate, and sodium methyl stearoyl taurate and mixtures thereof. Preferred are potassium cocoyl taurate, potassium methyl cocoyl taurate, sodium caproyl methyl taurate, sodium cocoyl taurate, sodium lauroyl taurate, sodium methyl cocoyl taurate and sodium methyl lauroyl taurate and mixtures thereof. More preferred are potassium cocoyl taurate, potassium methyl cocoyl taurate, sodium cocoyl taurate, sodium lauroyl taurate, sodium methyl cocoyl taurate and sodium methyl lauroyl taurate and mixtures thereof.

Suitable glutamate surfactants are according to the general formula

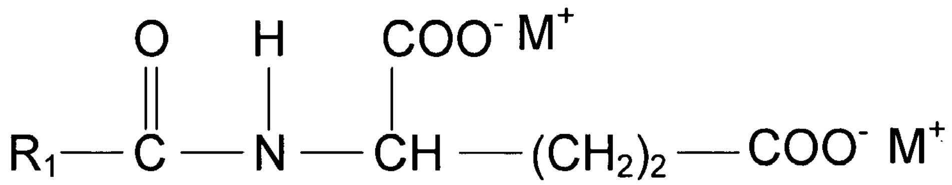

wherein $R_1$ is preferably a saturated or unsaturated, straight or branched alkyl chain with 7 to 17 C atoms, and more preferably 9 to 13 C atoms, and M is independent from each other H, sodium or potassium. Suitable examples are dipotassium capryloyl glutamate, dipotassium undecylenoyl glutamate, disodium capryloyl glutamate, disodium cocoyl glutamate, disodium lauroyl glutamate, disodium stearoyl glutamate, disodium undecylenoyl glutamate, potassium capryloyl glutamate, potassium cocoyl glutamate, potassium lauroyl glutamate, potassium myristoyl glutamate, potassium stearoyl glutamate, potassium undecylenoyl glutamate, sodium capryloyl glutamate, sodium cocoyl glutamate, sodium lauroyl glutamate, sodium myristoyl glutamate, sodium olivoyl glutamate, sodium palmitoyl glutamate, sodium stearoyl glutamate, and sodium undecylenoyl glutamate and mixtures thereof. Preferred are disodium capryloyl glutamate, disodium cocoyl glutamate, disodium lauroyl glutamate, potassium capryloyl glutamate, potassium cocoyl glutamate, potassium lauroyl glutamate, potassium myristoyl glutamate, sodium capryloyl glutamate, sodium cocoyl glutamate, sodium lauroyl glutamate, and sodium myristoyl glutamate and mixtures thereof. More preferred are disodium capryloyl glutamate, disodium cocoyl glutamate, disodium lauroyl glutamate, potassium capryloyl glutamate, potassium cocoyl glutamate, potassium lauroyl glutamate, sodium capryloyl glutamate, sodium cocoyl glutamate, and sodium lauroyl glutamate and mixtures thereof.

Suitable alanine or alaninate surfactants are according to the general formula

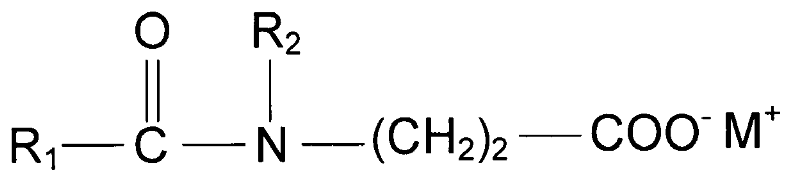

wherein $R_1$ is preferably a saturated or unsaturated, straight or branched alkyl chain with 7 to 17 C atoms, and more preferably 9 to 13 C atoms, $R_2$ is H or methyl and M is H, sodium or potassium. Suitable examples are cocoyl methyl β-alanine, lauroyl β-alanine, lauroyl methyl β-alanine, myristoyl β-alanine, potassium lauroyl methyl β-alanine, sodium cocoyl alaninate, sodium cocoyl methyl β-alanine and sodium myristoyl methyl β- alanine and mixtures thereof.

Suitable glycine surfactants are according to the general formula

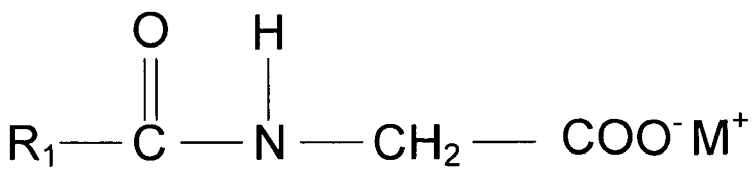

wherein $R_1$ is preferably a saturated or unsaturated, straight or branched alkyl chain with 7 to 17 C atoms, and more preferably 9 to 13 C atoms, and M is H, sodium or potassium. Suitable examples are palmitoyl glycine, sodium lauroyl glycine, sodium cocoyl glycine, sodium myristoyl glycine, potassium lauroyl glycine, and potassium cocoyl glycine and mixtures thereof.

Suitable sarcosinate surfactants are according to the general formula

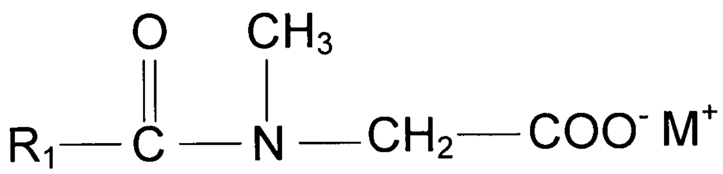

wherein $R_1$ is preferably a saturated or unsaturated, straight or branched alkyl chain with 7 to 17 C atoms, and more preferably 9 to 13 C atoms, and M is H, sodium or potassium. Suitable examples are potassium lauroyl sarcosinate, potassium cocoyl sarcosinate, sodium cocoyl sarcosinate, sodium lauroyl sarcosinate, sodium myristoyl sarcosinate, and sodium palmitoyl sarcosinate and mixtures thereof. Preferred are potassium lauroyl sarcosinate, potassium cocoyl sarcosinate, sodium cocoyl sarcosinate, and sodium lauroyl sarcosinate and mixtures thereof. More preferred are sodium cocoyl sarcosinate, and sodium lauroyl sarcosinate and mixtures thereof.

Suitable aspartate surfactants are according to the general formula

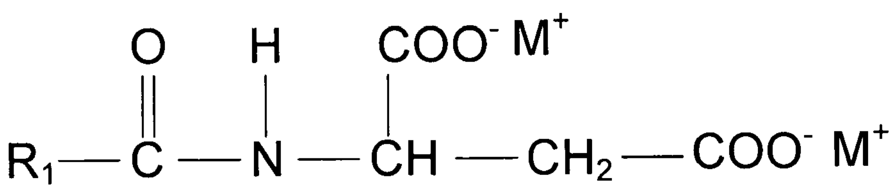

wherein $R_1$ is preferably a saturated or unsaturated, straight or branched alkyl chain with 7 to 17 C atoms, and more preferably 9 to 13 C atoms, and M is independent from each other H, sodium or potassium. Suitable examples are sodium lauroyl aspartate, sodium myristoyl aspartate, sodium cocoyl aspartate, sodium caproyl aspartate, disodium lauroyl aspartate, disodium myristoyl aspartate, disodium cocoyl aspartate, disodium caproyl aspartate, potassium lauroyl aspartate, potassium myristoyl aspartate, potassium cocoyl aspartate, potassium caproyl aspartate, dipotassium lauroyl aspartate, dipotassium myristoyl aspartate, dipotassium cocoyl aspartate, and dipotassium caproyl aspartate and mixtures thereof. Preferred are sodium lauroyl aspartate, sodium myristoyl aspartate, sodium cocoyl aspartate, and sodium caproyl aspartate and mixtures thereof.

It should be noted that compositions of the present invention can also comprise mixture of several type of amino acid surfactants such as mixture of glutamate and taurate surfactants, or mixture of taurate, glutamate and sarcosinate surfactants etc.

Cleansing compositions of the present invention comprise at least one anionic surfactant at a concentration range of 2 to 25%, preferably 2.5 to 20% and more preferably 5 to 15%, and most preferably 7.5 to 15% by weight, calculated to the total composition.

In principal any anionic surfactant is suitable within the meaning of the present invention. As mentioned above

with the term anionic surfactant any anionic surfactants are meant other than amino acid surfactants. Nonlimiting examples are anionic surfactants of the sulfate, sulfonate, carboxylate and alkyl phosphate type, especially, of course, those customarily used in shampoo compositions, for example, the known $C_{10}$-$C_{18}$-alkyl sulfates, and in particular the respective ether sulfates, for example, $C_{12}$-$C_{14}$-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono-and dialkyl phosphates constituting mild, skin-compatible detergents.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

$$R_5\text{-}(C_2H_4O)_n\text{-}O\text{-}CH_2COOX,$$

wherein $R_5$ is a $C_8$-$C_{20}$-alkyl group, preferably a $C_{12}$-$C_{14}$-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula

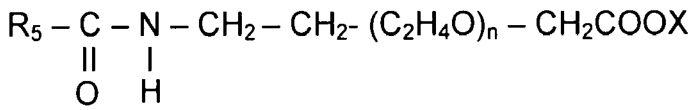

wherein $R_5$ and X have the above meanings, and n is in particular a number from 1 to 10, preferably 2.5 to 5.

Such products have been known for some time and are on the market, for example, under the trade name "AKYPO®" and "AKYPO-SOFT®".

Also useful are $C_8$-$C_{20}$-acyl isethionates, alone or in admixture with other anionic surfactants, as well as sulfofatty acids and the esters thereof.

It is also possible to use mixtures of several anionic surfactants, for example an ether sulfate and a polyether carboxylic acid or alkyl amidoether carboxylic acid.

The most preferred anionic surfactants within the meaning of the present invention are those of alkyl ether sulphates such as lauryl ether sulphate sodium salt.

In a preferred embodiment of the present invention, cleansing composition of the present invention comprises at least one anionic surfactant as mentioned above and at least one nonionic surfactant. Nonionic surfactants are suitable at a concentration of 1 % to 15 %, in particular from 1 % to 10 % by weight, calculated to the total composition.

Nonionic surfactants especially suited in the cleansing compositions according to the invention are alkyl polyglucosides of the general formula

$$R_6\text{-}O\text{-}(R_4O)_n\text{-}Z_x,$$

wherein $R_6$ is an alkyl group with 8 to 18 carbon atoms, $R_4$ is an ethylene or propylene group, Z is a saccharide group with 5 to 6 carbon atoms, n is a number from 0 to 10 and x is a number between 1 and 5.

These alkyl polyglucosides are known in particular as excellent skin-compatible, foam improving agents in liquid detergents and body cleansing compositions. Mixtures of anionic surfactants and alkyl polyglucosides as well as the use thereof in liquid body cleansing compositions are already known, for example, from EP-A 70 074. The alkyl polyglucosides disclosed therein are basically also suited within the scope of the present invention; as well as the mixtures of sulfosuccinates and alkyl polyglucosides disclosed in EP-A 358 216.

Further nonionic surfactants are, suitable for the cleansing compositions of the present invention, long-chain fatty acid dialkanolamides, such as coco fatty acid diethanolamide and myristic fatty acid diethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics$^R$", as well as fatty alcohol ethoxylates.

Further suitable nonionic surfactants are amineoxides which may be present in an amount from 0.25 % to 5 % by weight, calculated to the total composition. Such amineoxides are state of the art, for example $C_{12}$-$C_{18}$- alkyl dimethyl amineoxides such as lauryl dimethyl amineoxide, $C_{12}$-$C_{18}$- alkyl amidopropyl or -ethyl amineoxides, $C_{12}$-$C_{18}$ -alkyl di(hydroxyethyl) or (hydroxypropyl) amineoxides, or also amineoxides with ethyleneoxide and/or propyleneoxide

groups in the alkyl chain. Such amineoxides are on the market, for example, under the trade names "Ammonyx®", "Aromox®" or "Genaminox®".

Further nonionic surfactants useful in the compositions according to invention are $C_{10}$-$C_{22}$-fatty alcohol ethoxylates at a concentration of 0.5 to 10%, preferably 0.5 to 5% by weight, calculated to total composition. Especially suited are $C_{10}$-$C_{22}$-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

The most preferred non-ionic surfactants are alkyl polyglucosides such as decyl, cocoyl polyglucoside and ethoxylated fatty alcohols such as laureth-16.

In a further preferred embodiment of the present invention, cleansing composition of the present invention comprises at least one anionic, at least one nonionic surfactant and at least one amphoteric or zwitterionic surfactant.

Amphoteric or zwitterionic surfactants, are present at a concentration of 0.5 % to about 15 %, preferably 1 % to about 10 %, by weight, calculated to the total composition. It has especially been found out that addition of zwitterionic or amphoteric surfactants enhances foam feeling in terms of creaminess, foam volume and as well as skin compatibility are also improved. For achieving milder formulations anionic surfactant, especially of sulphate types, to amphoteric surfactant ratio should be in the range of 10:1 to 1:1, preferably 5:1 to 1:1.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, suitable betaine surfactants are of general structure

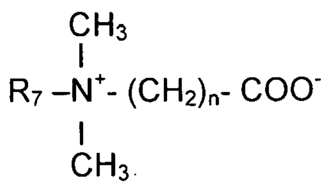

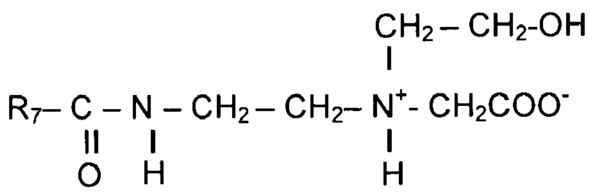

wherein $R_7$ is a $C_8$-$C_{18}$-alkyl group and n is 1 to 3;
sulfobetaines of the structure

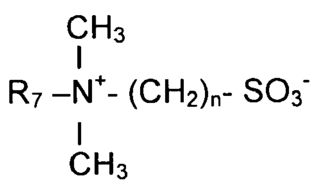

wherein $R_7$ and n are same as above;
and amidoalkyl betaines of the structure

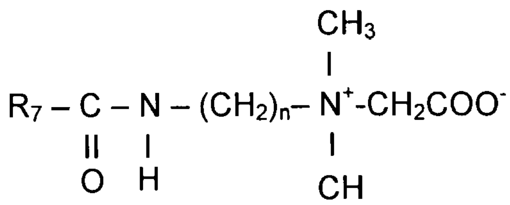

wherein $R_7$ and n are same as above.

The most preferred amphoteric surfactants are alkyl betaines such as lauryl betaine and alkyl amido betaines such as cocamidopropyl betaine.

In a further preferred form of the present invention, cleansing composition comprises at least one anionic surfactant especially of alkyl ether sulphate type, at least one amphoteric surfactant especially alkyl amido alkyl betaine type and at least one non-ionic surfactant especially an alkyl polyglucoside.

In another preferred form of the present invention cleansing compositions comprise additionally at least one (poly)propylene glycol according to the following formula

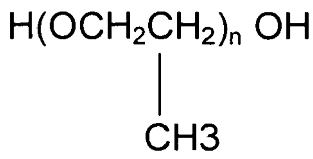

wherein n has a value between 1 and 70. It has been observed additionally that cleansing composition comprising at least one (poly)propylene glycol show excellent volume up effect especially for fine hair.

Non-limiting suitable examples are PPG-3, PPG-7, PPG-9, PPG-12, PPG-13, PPG-15, PPG-16, PPG-17, PPG-20, PPG-26, PPG-30, PPG-33, PPG-34, PPG-51 and PPG-69. Preferred are PPG-3, PPG-7, PPG-9, PPG-12, PPG-13, PPG-15, PPG-16, PPG-17, PPG-20, PPG-26, PPG-30, PPG-33 and PPG-34 and mixtures thereof. More preferred polypropylene glycols are PPG-7, PPG-9, PPG-12, PPG-13, PPG-15, PPG-16, PPG-17, PPG-20, PPG-26 and PPG-30 and mixtures thereof. Most preferred ones are PPG-7, PPG-9, PPG-12, PPG-13, PPG-15, PPG-16, PPG-17 and PPG-20 and mixtures thereof. Especially preferred one is PPG-9 with which most of the experiments were carried out.

Concentration of at least one (poly)propylene glycol of the above formula in compositions of the present invention is in the range of 0.1 to 10%, more preferably 0.25 to 7.5%, and most preferably 0.5 to 5% by weight, calculated to total composition.

In a further preferred embodiment, cleansing composition of the present invention comprises hair-conditioning agents. Conditioning agents can be selected from oily substances, non-ionic substances, cationic amphiphilic ingredients, cationic polymers or their mixtures.

Oily substances are selected from such as silicone oils, either volatile or non-volatile, natural and synthetic oils. Among silicone oils those can be added to the compositions include dimethicone, dimethiconol, polydimethylsiloxane, DC fluid ranges from Dow Corning, arylated silicones such as phenyl trimethicone or any other silicone with up to 5 aryl, preferably phenyl, group in its molecule, natural oils such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents can be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

$$R_8\,CO\,(O\,CH_2\,CH_2)_n\,OH$$

or

$$R_8\,CO\,(O\,CH_2\,CH_2)_n\,O\,OC\,R_9$$

where $R_8$ and $R_9$ are independent from each other saturated, unsaturated or branched or non-branched alkyl chain with

7 to 21 C atoms and n is typically 2 - 100.

In one of the preferred form of the present invention, cleansing compositions comprise at least one cationic polymer as conditioning agent. Suitable cationic polymers are those of best known with their CTFA category name Polyquaternium. Typical examples of those are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 and Quaternium-84.

It has further been found out that especially those of cationic cellulose type polymers known as Polymer JR type from Amerchol such as Polyquaternium 10 or cationic galactomannans such as cationic guar gum known with trade name Jaguar from Rhône-Poulenc which are chemically for example Guar hydroxypropyl trimonium chloride and cationic tara gum an its derivatives known with INCI name Caesalpinia spinosa hydroxypropyltrimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, cationic Caesalpinia spinosa gum derivatives, polyquaternium 6, polyquaternium 7, polyquaternium 67 and polyquaternium 70.

The cationic polymers also include the quaternized products of graft polymers from organopolysiloxanes and polyethyl oxazolines described in EP-A 524 612 and EP-A 640 643.

Although less preferred, cleansing compositions of the present invention may comprise additionally one or more cationic surfactant(s) as conditioner presented with the general formula

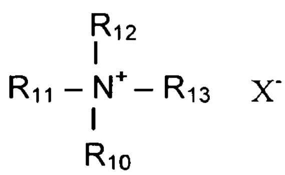

where $R_{10}$ is a saturated or unsaturated, branched or non-branched alkyl chain with 8-22 C atoms or

$R_{14}$ CO NH $(CH_2)_n$

where $R_{14}$ is saturated or unsaturated, branched or non-branched alkyl chain with 7-21 C atoms and n has value of 1 - 4, or

$R_{15}$ CO O $(CH_2)_n$

where $R_{15}$ is saturated or unsaturated, branched or non-branched alkyl chain with 7-21 C atoms and n has value of 1 - 4, and

$R_{11}$ is hydrogen or unsaturated or saturated, branched or non-branched alkyl chain with 1 - 22 C atoms or

$R_{14}$ CO NH $(CH_2)_n$

or

$R_{15}$ CO O $(CH_2)_n$

where $R_{14}$ , $R_{15}$ and n are same as above.

$R_{12}$ and $R_{13}$ are hydrogen or lower alkyl chain with 1 to 4 carbon atoms, and X is anion such as chloride, bromide, methosulfate.

Typical examples of those ingredients are cetyl trimethly ammonium chloride, stear trimonium chloride, behentrimonium chloride, stearamidopropyl trimonuim chloride, dioleoylethyl dimethyl ammonium methosulfate, dioleoylethyl hydroxyethylmonium methosulfate.

The compositions according to the invention may also comprise further conditioning substances such as protein hydrolyzates and polypeptides, e.g., keratin hydrolyzates, collagen hydrolyzates of the type "Nutrilan$^R$" or elastin hydrolyzates, as well as also in particular plant protein hydrolyzates, optionally, cationized protein hydrolyzates, e.g., "Gluadin$^R$".

Typical concentration range for any of those conditioners of cationic polymers, silicon oil and derivatives and cationic surfactants can be 0.01 - 5% by weight, preferably 0.01 - 3.5% by weight, more preferably 0.05 - 2.5% and most preferably 0.1 - 1.5% by weight calculated to the total composition. Most preferred conditioning agents are cationic polymers.

Further conditioning additives are hair conditioning and/or styling polymers. These may be nonionic polymers, preferably alcohol- and/or water-soluble vinyl pyrrolidone polymers, such as a vinyl pyrrolidone homopolymers or copolymers, in particular with vinyl acetate. Useful vinyl pyrrolidone polymers are, e.g., those known by the trade name "Luviskol®", for example, the homopolymers "Luviskol® K 30, K 60 and K 90", as well as the water-or alcohol-soluble copolymers from vinyl pyrrolidone and vinyl acetate, distributed by BASF AG under the trade name "Luviskol® VA 55 respectively VA 64". Further possible nonionic polymers are vinyl pyrrolidone/vinyl acetate/vinyl propionate copolymers such as "Luviskol® VAP 343", vinyl pyrrolidone/(meth)acrylic acid ester copolymers, as well as chitosan derivatives.

Amphoteric polymers are found to be useful in conditioning shampoo composition of the present invention. They are incorporated alone or in admixture with at least one additional cationic, nonionic or anionic polymer, particularly copolymers of N-octyl acrylamide, (meth)acrylic acid and tert.-butyl aminoethyl methacrylate of the type "Amphomer®"; copolymers from methacryl oylethyl betaine and alkyl methacrylates of the type "Yukaformer®", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl amino alkyl(meth)acrylates or mono- or dialkyl aminoalkyl (meth)acrylamides containing basic groups, in particular amino groups; copolymers from N-octyl acrylamide, methyl methacrylate, hydroxypropyl methacrylate, N-tert.-butyl aminoethyl - methacrylate and acrylic acid, as well as the copolymers known from US-A 3,927,199, are applicable.

Cleansing composition of the present invention can be transparent as well as pearly. Transparency of the composition is judged by naked eye in a transparent shampoo bottle with a thickness not more than 5 cm. In the case a transparent appearance is wished, the following ingredients are not essential. Pearl-shiny appearance is achieved with those dispersed in cleansing conditioning compositions in crystalline form, i.e. so called pearl-shine or pearlizing agents. The preferred once are PEG-3 distearate and ethylene glycol distearate. The concentration of those can typically be from 0.1 to 3%, preferably 0.5 to 2% by weight, calculated to the total composition. These compounds are preferably added to the compositions in admixture with anionic, nonionic and/or amphoteric surfactants. Such kinds of mixtures are available commercially.

Solubilizers may be added to the compositions especially when oily substances are chosen as conditioning agents and fragrance oils with highly lipophilic properties. Typical solubilizers may be hydrogenated castor oil known with the trade mark Cremophor CO series from BASF. It should be noted that as well the surfactant mixture can be a good solubilizer for fragrance oils. Typical concentration of the solubilizers can be in the range of 0.01 - 2% by weight, preferably 0.1 - 1 % by weight, calculated to total composition.

The cleansing composition may contain active ingredients selected from UV filters, moisturisers, sequestering agents, and natural ingredients.

The moisturizing agents are selected from panthenol, polyols, such as glycerol, polyethylene glycols with molecular weight 200 to 20,000. The moisturizing ingredients can be included in the conditioner compositions at a concentration range of 0.01 - 2.5% by weight calculated to the total composition.

The sequestering agents are preferably selected from polycarboxy acids. The preferred one is ethylene diamine tetraacetic acid, EDTA. Typical useful concentration range for sequestering agents is of 0.01 - 2.5% by weight calculated to the total composition.

The UV filters are that oil and water soluble ones for the purpose of protecting hair colour. In other words, anionic and nonionic, oily, UV filters are suitably used in the compositions of the present invention. Suitable UV-absorbing

substances is are: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxybenzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher, and/or polysilicone-15. The amount of the UV-absorber ranges typically from about 0.01 % to 2.5%, more preferably from 0.05 % to 1 % by weight, calculated to the total composition.

Natural plant extracts are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, various "Extrapone®" products, and "Herbasol$^R$ ". Extracts and the preparation thereof are also described in "Hagers Handbuch der pharmazeutischen Praxis", 4th Ed..

Compositions of the present invention may comprise further at least one compound according to the formula

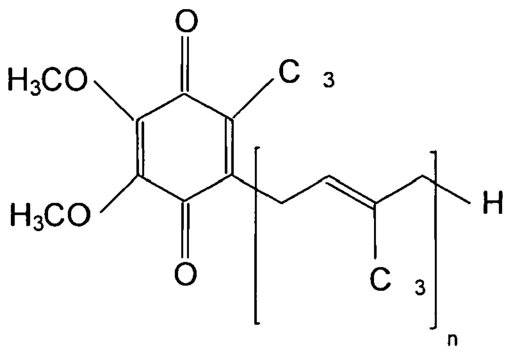

where n is a number from 1 to 10.

The compounds of the above formula are known as Ubiquinone, and also are known as Coenzyme. It should be noted that the compositions of the present invention can certainly comprise more than one ubichinone. Preferred ubichinones are the ones

where n is a number between 6 and 10 and especially preferred is Ubichinone 50

where n is 10, also known as Coenzyme Q10. Concentration ubichinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

Cleansing compositions of the present invention can also comprise synthetic mica as a further shine enhancer.

Use of synthetic mica coated with metal oxide or oxides mainly in decorative cosmetics is disclosed in an international patent application of Sun Chemical Corporation published with a number WO 2005/065632 A1. In the document synthetic mica and coated synthetic mica with at least one metal oxide or oxides is disclosed in detail, the content of the document is included herewith by reference. It also discloses a cleansing composition comprising monoethanolamide surfactant in addition to other surfactants.

Suitable metal oxide or oxides for coating synthetic mica are titanium dioxide, chromium oxide, ferric oxide or mixtures thereof. In the present invention the preferred is synthetic mice coated with titanium dioxide. Such materials are commercially available from Sun Chemical Corporation and known with their INCI names Synthetic Fluorphologopite.

The particle size distribution of synthetic mica coated with a metal oxide or oxides is in the range of 1 to 750 μm, preferably 1 to 250 μm, more preferably 1 to 100 μm and most preferably 20 to 95 μm. The particle sizes referred are relating to the volume particle size distribution meaning that particles found in the coated synthetic mica having volume particle size in the given ranges.

Concentration of synthetic mica coated with at least metal oxide or oxides is from 0.001 to 10%, preferably

0.05 to 7.5%, more preferably 0.1 to 5% and most preferably 0.20 to 2.5% by weight calculated to total composition.

Further in a preferred embodiment of the present invention, compositions comprise at least one direct dye. Suitable direct dyes are of cationic, anionic and neutral nitro dyes. It should be noted that they can also be used in combination with each other. In other words a composition according to present invention can comprise an anionic and a cationic dye as well as an anionic and a nitro dye or a cationic and a nitro dye. Certainly the combination of all three dyestuff categories is also possible.

Any cationic direct dye is in principal suitable for the compositions. Examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87, and mixtures thereof

Any anionic dye is in principal suitable for the compositions. Suitable examples are such as Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium, and mixtures thereof.

Among those, the preferred anionic dyestuffs are Acid Red 52, Acid Violet 2, Acid Red 33, Acid Orange 4, Acid Red 27 and Acid Yellow 10 and their salts, and mixtures thereof. The most preferred anionic dyes are Acid Red 52, Acid Violet 2, Acid Red 33, Acid Orange 4 and Acid Yellow 10, and their salts, and mixtures thereof.

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No. 1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No. 11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid, and mixtures thereof.

Concentration of one or more direct dyes in total is in the range of 0.001 to 5% by weight, preferably 0.01 to 4% more preferably 0.05 to 3% and most preferably 0.1 to 2.5% by weight calculated to total composition. The most preferred among the direct dyes is cationic direct dyes.

It is self-understood that the shampoos according to the invention may comprise other substances customarily used in such compositions such as preservatives, fragrances.

The pH of the compositions according to the present invention is suitably between 2 and 8.0, preferably in the range of 2.5 to 7.0, more preferably 3 to 6.5 and most preferably 4 to 5.5 measured at ambient temperature with a suitable pH meter.

pH of the compositions is adjusted with acidic and alkaline compounds. Acidic compounds can be inorganic and organic acid or their mixtures. Nonlimiting suitable examples are citric acid, lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid. Alkaline compounds such as sodium hydroxide can be used to adjust the pH of the compositions.

Cleansing compositions of the present invention preferably has a viscosity value at zero or very low shear stress, due to viscosity measuring conditions, and at 20°C measured with Brookfield viscosimetre in the range of 1,000 to 60,000, preferably 2,000 to 50,000 and more preferably 5,000 to 40,000 mPa.s.

The following examples are to illustrate the invention, but not to limit. The products according to the invention are prepared by mixing the individual components in water, whereby it is also possible to use pre-mixtures of various ingredients.

**Example 1**

| | % by weight |
|---|---|
| Sodium lauryl ether sulphate | 12.6 |
| Sodium lauroyl glutamate | 1.5 |
| PEG-90 glyceryl isostearate | 3.0 |
| Citric acid/sodium hydroxide | q.s. to pH 5.5 |
| Preservative, fragrance | q.s |
| Water | to 100 |

For the comparative purposes the above composition was also prepared without PEG-90 glyceryl isostearate. In the comparative example 2% by weight sodium chloride is used as thickening agent. Viscosity of the both compositions measured at 20°C with a Brookfield viscosimetre with Spindle 93 at 5 rpm were approximately 35,000 mPa.s (35 Pa.s).

Rheopex behaviour of the cleansing compositions is determined according to the method disclosed in "Das Rheologie Handbuch, 2. Auflage, Menzer, T.G., Vincentz Network GmbH, Hannover, 2006, pages 246 - 250" at 20°C.

Briefly, the above compositions were filled into a cylinder shaped "syringe" like vessel with a diameter of 0.025 m (d) and with an opening diameter of 0.006 m ($d_3$). The composition filled into the vessel had a volume of 2.45 x $10^{-5}$ $m^3$ (V) and a height of 0.05 m ($L_1$). The height of opening was 0.02 m ($L_3$). Under a weight of 0.545 kg (m), the flow time for the complete emptying of the cleansing composition from the vessel was determined in seconds ($t_i$ = inventive composition and $t_c$ = comparative composition). The emptying times determined were $t_i$ = 77 s and $t_c$ = 12 s). With the help of the equations below the viscosity values were calculated in Pa.s.

$$F = m * g$$

wherein F is the force in N and m is the weight in kg and g is the gravity constant (9.8 m $s^{-2}$).

From the above equation a force of 5.35 N is calculated.

The pressure (p) at the opening was calculated with the following equation:

$$p = \frac{F}{A} = \frac{F}{r^2 \pi} = pressure$$

wherein p is pressure, A is the cross sectional area of the vessel in $m^2$ wherein the compositions were filled and r is radius in m.

The shear stress ($\tau$) at the opening was calculated with the following equation:

$$\tau = \frac{p * R_3}{2 * L_3} = \frac{F * R_3}{2 * L_3 * \left(\frac{d}{2}\right)^2 * \pi} = \frac{F * d_3}{L_3 * d^2 * \pi}$$

wherein $R_3$ is radius at the opening in m.

Form the above equation under the current measurement conditions, the value of $\tau$ was obtained to be 817.3 $Nm^{-2}$

The shear rate ($\gamma$) at the opening was calculated with the following equation:

$$\dot{\gamma} = \frac{4 * \dot{V}}{\pi * R_3^3} = \frac{4 * V}{\pi * R_3^3 * t}$$

Form the above equation, $\gamma$ value for the inventive composition ($\gamma_i$) with $t_i$ value of 77 s at the place of t was

calculated to be 96.3 $s^{-1}$ and for the comparative composition ($\gamma_c$) with $t_c$ value of 12 s at the place of t was calculated to be 15.0 $s^{-1}$ .

Viscosity values ($\eta$) during the shear stress are calculated using the following equation:

$$\eta = \frac{\tau}{\dot{\gamma}} = \left[\frac{Ns}{m^2} = Pas\right]$$

With the above mentioned $\tau$ and $\gamma_i$ and $\gamma_c$ values calculated for inventive composition and for comparative composition, respectively, the following viscosity values for inventive composition ($\eta_i$) and comparative composition ($\eta_c$) were calculated:

$\eta_I$ = 54.4 Pa.s

$\eta_c$ = 8.5 Pa.s

From the above it is clear that the inventive composition is a shear thickening composition, whereas the comparative composition is a shear thinning composition when for both compositions viscosity values measured with Brookfield viscosimetre (35 Pa.s) are taken into account with practically zero or negligibly very low shear stress wneh compared to the values used above.

Similar results were observed with the examples below.

**Example 2**

| | % by weight |
|---|---|
| Sodium lauryl ether sulphate | 9.0 |
| Cocyl glucoside | 4.0 |
| Sodium lauroyl glutamate | 2.0 |
| Polyquaternium-7 | 1.0 |
| PEG-90 glyceryl isostearate | 2.0 |
| Citric acid/sodium hydroxide | q.s. to pH 5.5 |
| Preservative, fragrance | q.s |
| Water | to 100 |

The above composition has excellent creamy rich foam and conditions hair excellently in terms of combability and soft hair feeling.

**Example 3**

| | % by weight |
|---|---|
| Sodium lauryl ether sulfate | 9.0 |
| Cocyl glucoside | 3.0 |
| Cocoyl betaine | 2.0 |
| Sodium lauroyl glutamate | 2.0 |
| Polyquaternium-6 | 0.5 |
| Dimethicone | 0.5 |
| Ubiquinone | 0.1 |
| PEG-90 glyceryl isostearate | 3.0 |
| PPG-9 | 2.0 |
| Citric acid/sodium hydroxide | q.s. to pH 5.0 |
| Preservative, fragrance | q.s |

(continued)

| | % by weight |
|---|---|
| Water | to 100 |

The above composition improves hair volume, gives hair more elasticity in addition to the excellent creamy foam and conditioning effect in terms of combability, shine and soft hair feeling.

The above composition was tested with 10 female users. For comparative purposes, a composition not comprising PEG-90 gylceryl isosterate was prepared which was thickened with 2% sodium chloride. Both compositions had a viscosity of approximately 20,000 mPa.s measured with Brookfield viscosimetre at 20°C. Both compositions were filled in a tube with an opening diameter of 2 mm. The volunteers were asked to get out enough amount of shampoo from the tube for one hair wash as they usually do and pay attention to the consistency. All 10 volunteers preferred the consistency of the inventive composition because of its thicker consistency and gel like behaviour when freshly squeezed from the tube. Furthermore, it was found to be interesting that the shampoo composition became less thick and spreads on the hand surface. Comparative composition was found to be spreading on hand too quickly. At the same time, all volunteers said that the composition is slightly more difficult to get out from the tube compared to the comparative composition. This shows clearly that under the same condition, inventive composition thickens when taken out from a tube with a small opening diameter.

**Example 4**

| | % by weight |
|---|---|
| Sodium lauryl ether sulphate | 8.0 |
| Cocoyl polyglucoside | 1.5 |
| Cocamidopropyl betaine | 4.0 |
| Sodium cocoyl glutamate | 2.0 |
| Polyquaternium-7 | 0.8 |
| Dimethicone | 0.5 |
| PEG-90 glyceryl isostearate | 5.0 |
| PPG-9 | 1.2 |
| Basic red 51 | 0.1 |
| Citric acid/sodium hydroxide | q.s. to pH 5.5 |
| Preservative, fragrance | q.s |
| Water | to 100 |

The above composition gives hair a red shine, and additionally delivers excellent conditioning effect in terms of more elasticity, combability, shine and soft hair feeling in addition to the excellent creamy foam.

**Example 5**

| | % by weight |
|---|---|
| Sodium lauryl ether sulfate | 9.0 |
| Laureth - 16 | 3.0 |
| Cocoyl betaine | 2.0 |
| Sodium cocoyl glutamate | 2.0 |
| Guarhydroxypropyltrimonium chloride | 1.0 |
| PEG-90 glyceryl isostearate | 2.0 |
| PPG-9 | 1.0 |
| Trimethyl pentaphenyl trisiloxane | 0.3 |
| Basic yellow 87 | 0.08 |
| Basic red 76 | 0.001 |

(continued)

| | % by weight |
|---|---|
| Citric acid/sodium hydroxide | q.s. to pH 5.0 |
| Preservative, fragrance | q.s |
| Water | to 100 |

Excellent conditioning effects were observed in terms of volume, combability, elasticity and managabiliy and additionally an excellent golden blonde shine was observed on light blond hair.

**Example 6**

| | % by weight |
|---|---|
| Sodium lauryl ether sulfate | 9.0 |
| Cocyl glucoside | 3.0 |
| Lauryl betaine | 2.0 |
| Sodium cocoyl glutamate | 2.0 |
| Guarhydroxypropyltrimonium chloride | 1.0 |
| PEG-90 glyceryl isostearate | 2.0 |
| PPG-9 | 1.0 |
| Trimethyl pentaphenyl trisiloxane | 0.3 |
| Basic red 51 | 0.1 |
| Basic orange 31 | 0.05 |
| Citric acid/sodium hydroxide | q.s. to pH 5.0 |
| Preservative, fragrance | q.s |
| Water | to 100 |

Excellent red shine were observed on medium blond hair.

**Example 7**

| | % by weight |
|---|---|
| Sodium lauryl ether sulfate | 9.0 |
| Laureth - 16 | 3.0 |
| Cocoyl betaine | 2.0 |
| Sodium cocoyl glutamate | 2.0 |
| Polyquaternium-10 | 1.0 |
| PEG-90 glyceryl isostearate | 3.5 |
| PPG-9 | 0.7 |
| Carbopol Aqua CC | 5.0 |
| Synthetic fluorphologopite* | 0.5 |
| Citric acid/sodium hydroxide | q.s. to pH 4.7 |
| Preservative, fragrance | q.s |
| Water | to 100 |

*: Synthetic fluorphologopite used is commercially available from Sun Chemical Corporation under the trade name SunShine Glitter White with a particle size distribution in the range of 20 to 95 μm.

The above composition delivered excellent volume and shine to dark blonde fine hair.

**Example 8**

| | % by weight |
|---|---|
| Sodium lauryl ether sulfate | 10.0 |
| Cocoyl betaine | 2.0 |
| Decyl glucoside | 1.5 |
| Sodium lauroyl glutamate | 4.0 |
| Quaternium 80 | 0.5 |
| Polyquaternium-7 | 0.2 |
| PEG-90 glyceryl isostearate | 3.5 |
| PPG-9 | 1.7 |
| Citric acid/sodium hydroxide | q.s. to pH 5.0 |
| Preservative, fragrance | q.s |
| Water | to 100 |

Above shampoo was found to be excellent volume giving shampoo to fine hair in a monadic test.

**Example 9**

| | % by weight |
|---|---|
| Sodium lauryl ether sulphate | 8.0 |
| Cocoyl polyglucoside | 2.0 |
| Cocamidopropyl betaine | 4.0 |
| Sodium cocoyl glutamate | 2.0 |
| Trimethyl pentaphenyl trisiloxane | 0.3 |
| Polyquaternium-7 | 1.0 |
| PEG-120 glyceryl stearate | 3.0 |
| PPG-15 | 1.7 |
| Citric acid/sodium hydroxide | q.s. to pH 5.2 |
| Preservative, fragrance | q.s |
| Water | to 100 |

Above shampoo was found to be excellent in volume enhancing effect. Additionally it improves combability and showed excellent shine enhancing effect.

**Example 10**

| | % by weight |
|---|---|
| Sodium lauryl ether sulphate | 8.0 |
| Cocoyl glucoside | 5.0 |
| Cocamidopropyl betaine | 4.0 |
| Sodium cocoyl glutamate | 2.0 |
| Polyquaternium-7 | 1.0 |
| PEG-90 glyceryl isostearate | 1.5 |
| PEG-30 glyceryl isostearate | 1.5 |
| PPG-15 | 0.3 |

(continued)

| | % by weight |
|---|---|
| PPG-9 | 0.8 |
| Citric acid/sodium hydroxide | q.s. to pH 5.2 |
| Preservative, fragrance | q.s |
| Water | to 100 |

The above composition increases hair volume, improves combability and shine.

**Example 11**

| | % by weight |
|---|---|
| Sodium lauryl ether sulphate | 5.0 |
| Sodium lauryl ether carboxylate | 3.0 |
| Laureth - 16 | 3.0 |
| Cocoyl betaine | 2.0 |
| Sodium lauroyl glutamate | 2.0 |
| Polyquaternium-6 | 0.5 |
| Dimethicone | 0.5 |
| Ubiquinone | 0.1 |
| PEG-60 glyceryl isostearate | 4.0 |
| PPG-9 | 2.0 |
| Citric acid/sodium hydroxide | q.s. to pH 5.0 |
| Preservative, fragrance | q.s |
| Water | to 100 |

The above shampoo conditions hair excellently in terms of combability, softness and elasticity and additionally gives fine hair excellent long lasting volume.

**Example 12**

| | % by weight |
|---|---|
| Sodium lauryl ether sulphate | 3.0 |
| Sodium lauryl ether carboxylate | 6.0 |
| Cocoyl polyglucoside | 3.0 |
| Cocoamphoacetate | 4.0 |
| Sodium cocyl glutamate | 2.0 |
| Cocoyl betaine | 1.0 |
| Polyquaternium-7 | 0.8 |
| Dimethicone | 0.5 |
| PEG-90 glyceryl isostearate | 3.0 |
| PPG- 12 | 0.6 |
| PPG-7 | 0.9 |
| Basic red 51 | 0.1 |
| Citric acid/sodium hydroxide | q.s. to pH 5.5 |
| Preservative, fragrance | q.s |
| Water | to 100 |

Above composition gives hair a red shine, in addition to the excellent conditioning effect.

**Example 13**

| | % by weight |
|---|---|
| Sodium lauryl ether sulfate | 9.0 |
| Laureth - 16 | 3.0 |
| Cocoyl betaine | 2.0 |
| Sodium lauroyl glutamate | 2.0 |
| Guarhydroxypropyltrimonium chloride | 1.0 |
| PEG-80 glyceryl cocoate | 4.0 |
| PPG-20 | 0.8 |
| Trimethyl pentaphenyl trisiloxane | 0.2 |
| Basic yellow 87 | 0.10 |
| Basic red 76 | 0.01 |
| Citric acid/sodium hydroxide | q.s. to pH 6.0 |
| Preservative, fragrance | q.s |
| Water | to 100 |

Increase of volume and an excellent golden blonde shine was observed on light blond hair. Conditioning effect in terms of manageability and soft feeling upon touching is excellent.

**Example 14**

| | % by weight |
|---|---|
| Sodium lauryl ether sulfate | 3.0 |
| Sodium lauryl ether carboxylate | 7.0 |
| Laureth - 16 | 3.0 |
| Cocoyl betaine | 2.0 |
| Sodium lauroyl glutamate | 2.0 |
| Guarhydroxypropyltrimonium chloride | 1.0 |
| PEG-120 glyceryl stearate | 1.8 |
| PPG-7 | 1.8 |
| Dimethicone | 1.0 |
| Basic red 51 | 0.1 |
| Basic orange 31 | 0.05 |
| Citric acid/sodium hydroxide | q.s. to pH 5.7 |
| Preservative, fragrance | q.s |
| Water | to 100 |

An excellent red shine were observed on medium blond hair.

**Example 15**

| | % by weight |
|---|---|
| Sodium lauryl ether sulphate | 10.0 |
| Laureth - 16 | 3.0 |

(continued)

| | % by weight |
|---|---|
| Cocoyl betaine | 2.0 |
| Sodium lauroyl glutamate | 2.0 |
| Polyquaternium-6 | 0.5 |
| Polysilicone-15 | 0.35 |
| Dimethicone | 0.5 |
| Ubiquinone | 0.1 |
| PEG-60 glyceryl isostearate | 4.0 |
| PPG-9 | 2.0 |
| Citric acid/sodium hydroxide | q.s. to pH 4.8 |
| Preservative, fragrance | q.s |
| Water | to 100 |

The above shampoo conditions hair excellently in terms of combability, softness, shine and elasticity and additionally gives fine hair excellent long lasting volume.

**Example 16**

| | % by weight |
|---|---|
| Sodium lauryl ether sulphate | 6.0 |
| Sodium lauryl ether carboxylate | 4.0 |
| Cocoyl glucoside | 3.0 |
| Cocamido propylbetaine | 2.0 |
| Sodium lauroyl glutamate | 2.0 |
| Polyquaternium-6 | 0.5 |
| Benzophenone-4 | 0.5 |
| Dimethicone | 0.5 |
| Ubiquinone | 0.1 |
| PEG-60 glyceryl isostearate | 4.0 |
| PPG-9 | 2.0 |
| Citric acid/sodium hydroxide | q.s. to pH 5.5 |
| Preservative, fragrance | q.s |
| Water | to 100 |

The above shampoo conditions hair excellently in terms of combability, shine, softness and elasticity and additionally gives fine hair excellent long lasting volume.

**Example 17**

| | % by weight |
|---|---|
| Sodium lauryl ether sulphate | 15.0 |
| Decyl glucoside | 3.0 |
| Cocamidopropyl betaine | 2.0 |
| Sodium cocyl glutamate | 2.0 |
| Polyquaternium-6 | 0.5 |
| Ethylhexyl methoxy cinnamate | 0.3 |
| Dimethicone | 0.5 |
| Ubiquinone | 0.1 |

(continued)

| | % by weight |
|---|---|
| PEG-60 glyceryl isostearate | 4.0 |
| PPG-9 | 2.0 |
| Citric acid/sodium hydroxide | q.s. to pH 5.2 |
| Preservative, fragrance | q.s |
| Water | to 100 |

**Example 18**

| | % by weight |
|---|---|
| Sodium lauryl ether sulphate | 10.0 |
| Cocyl glucoside | 5.0 |
| Lauryl betaine | 4.0 |
| Sodium lauroyl glutamate | 2.0 |
| Polyquaternium-10 | 0.5 |
| Benzophenone-3 | 0.4 |
| Dimethicone | 0.5 |
| PEG-60 glyceryl isostearate | 4.0 |
| PPG-9 | 2.0 |
| Citric acid/sodium hydroxide | q.s. to pH 5.4 |
| Preservative, fragrance | q.s |
| Water | to 100 |

## Claims

1. Cleansing composition for keratin fibres especially for human hair based on at least one anionic surfactant, especially anionic sulphate surfactant, at a concentration of 2 to 25% by weight, and further comprising at least one amino acid surfactant of the following structure

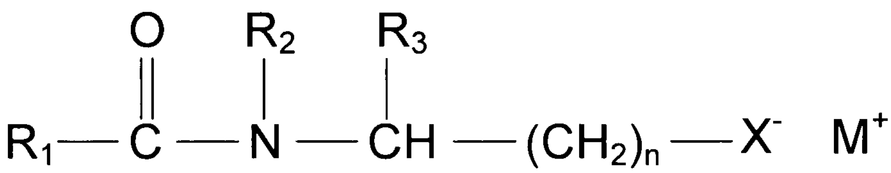

wherein $R_1$ is a saturated or unsaturated, straight or branched alkyl chain with 7 to 17 C atoms, $R_2$ is H or a methyl, $R_3$ is H, $COO^- M^+$, $CH_2COO^-$ M or COOH, n is 0 to 2, X is $COO^-$ or $SO_3^-$ and M is independent from each other H, sodium or potassium, at a concentration of 0.1 to 15%, by weight, and at least one ethoxylated monoglyceride of the following formula

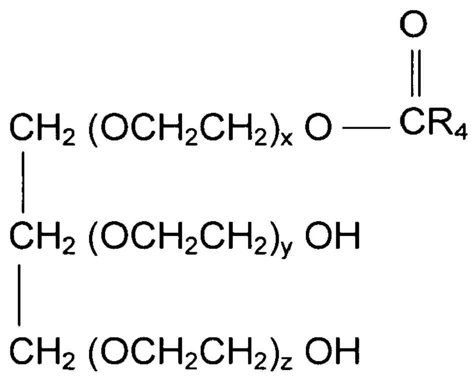

wherein $R_4$ is a saturated or unsaturated and branched or straight alkyl chain with a chain length of 7 to 21 C atoms and x+y+z has a value of 50 to 200, at a concentration of at least 1% by weight, all values calculated to total composition.

2. Cleansing composition according to claim 1 **characterised in that** it comprises additionally at least one non-ionic surfactant.

3. Cleansing composition according to claim 2 **characterised in that** it comprises at least one non-ionic surfactant according to the general formula

$$R_6\text{-O-}(R_4O)_n\text{-}Z_x,$$

wherein $R_6$ is an alkyl group with 8 to 18 carbon atoms, $R_4$ is an ethylene or propylene group, Z is a saccharide group with 5 to 6 carbon atoms, n is a number from 0 to 10 and x is a number between 1 and 5.

4. Cleansing composition according to any of the preceding claims **characterised in that** it comprises additionally at least one amphoteric surfactant.

5. Cleansing composition according to claim 4 **characterised in that** at least one amphoteric surfactant is selected from betaines, amidoalkyl betaines and sulfobetaines, and their mixtures.

6. Composition according to any of the proceedings claims **characterised in that** at least one amino acid surfactant is selected from

i- taurate surfactants according to the general formula

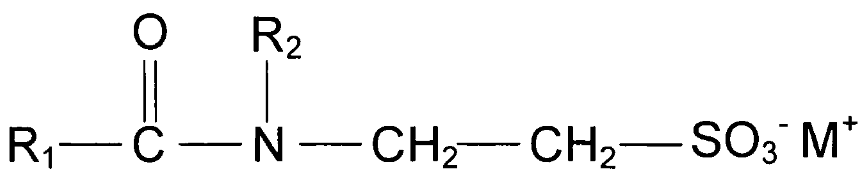

wherein $R_1$ is preferably a saturated or unsaturated, straight or branched alkyl chain with 7 to 17 C atoms, and more preferably 9 to 13 C atoms, $R_2$ is H or methyl, and M is H, sodium or potassium,
ii- glutamate surfactants are according to the general formula

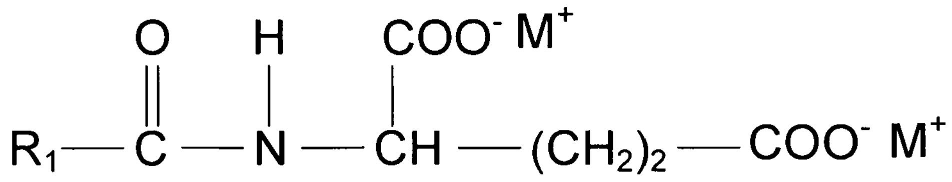

wherein $R_1$ is preferably a saturated or unsaturated, straight or branched alkyl chain with 7 to 17 C atoms, and more preferably 9 to 13 C atoms, and M is independent from each other H, sodium or potassium,
iii- alanine or alaninate surfactants according to the general formula

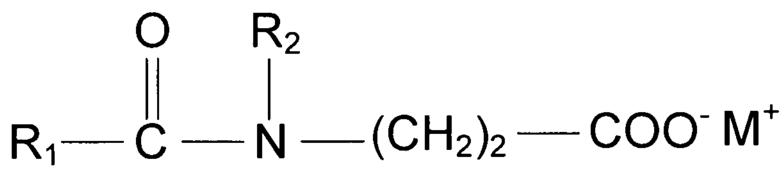

wherein $R_1$ is preferably a saturated or unsaturated, straight or branched alkyl chain with 7 to 17 C atoms, and more preferably 9 to 13 C atoms, $R_2$ is H or methyl and M is H, sodium or potassium,
iv- glycine surfactants according to the general formula

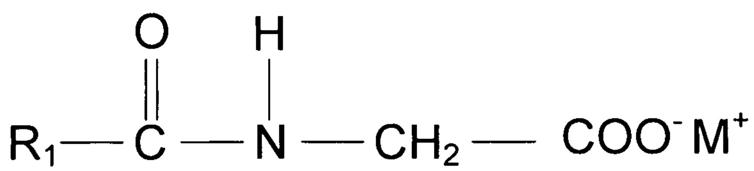

wherein $R_1$ is preferably a saturated or unsaturated, straight or branched alkyl chain with 7 to 17 C atoms, and more preferably 9 to 13 C atoms, and M is H, sodium or potassium,
v- sarcosinate surfactants according to the general formula

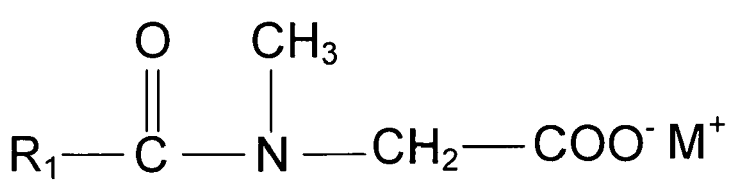

wherein $R_1$ is preferably a saturated or unsaturated, straight or branched alkyl chain with 7 to 17 C atoms, and more preferably 9 to 13 C atoms and M is H, sodium or potassium, and
vi- aspartate surfactants according to the general formula

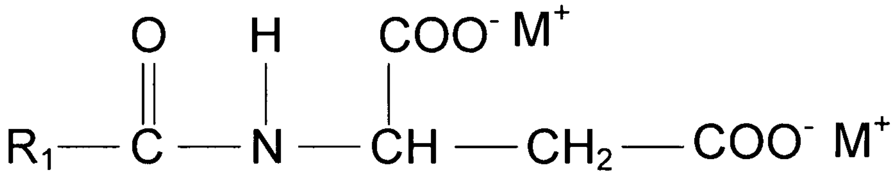

wherein $R_1$ is preferably a saturated or unsaturated, straight or branched alkyl chain with 7 to 17 C atoms, and more preferably 9 to 13 C atoms and M is independent from each other H, sodium or potassium.

7. Cleansing composition according to claim 6 **characterised in that** at least one amino acid surfactant is selected from potassium cocoyl taurate, potassium methyl cocoyl taurate, sodium caproyl methyl taurate, sodium cocoyl taurate, sodium lauroyl taurate, sodium methyl cocoyl taurate, sodium methyl lauroyl taurate, sodium methyl myristoyl taurate, sodium methyl oleoyl taurate, sodium methyl palmitoyl taurate, sodium methyl stearoyl taurate, dipotassium capryloyl glutamate, dipotassium undecylenoyl glutamate, disodium capryloyl glutamate, disodium cocoyl glutamate, disodium lauroyl glutamate, disodium stearoyl glutamate, disodium undecylenoyl glutamate, potassium capryloyl glutamate, potassium cocoyl glutamate, potassium lauroyl glutamate, potassium myristoyl glutamate, potassium stearoyl glutamate, potassium undecylenoyl glutamate, sodium capryloyl glutamate, sodium cocoyl glutamate, sodium lauroyl glutamate, sodium myristoyl glutamate, sodium olivoyl glutamate, sodium palmitoyl glutamate, sodium stearoyl glutamate, sodium undecylenoyl glutamate, cocoyl methyl β-alanine, lauroyl β-alanine, lauroyl methyl β-alanine, myristoyl β-alanine, potassium lauroyl methyl β-alanine, sodium cocoyl alaninate, sodium cocoyl methyl β-alanine and sodium myristoyl methyl β-alanine palmitoyl glycine, sodium lauroyl glycine, sodium cocoyl glycine, sodium myristoyl glycine, potassium lauroyl glycine, potassium cocoyl glycine, potassium lauroyl sarcosinate, potassium cocoyl sarcosinate, sodium cocoyl sarcosinate, sodium lauroyl sarcosinate, sodium myristoyl sarcosinate, and sodium palmitoyl sarcosinate and mixtures thereof. Preferred are potassium lauroyl sarcosinate, potassium cocoyl sarcosinate, sodium cocoyl sarcosinate, sodium lauroyl sarcosinate, sodium lauroyl aspartate, sodium myristoyl aspartate, sodium cocoyl aspartate, sodium caproyl aspartate, disodium lauroyl aspartate, disodium myristoyl aspartate, disodium cocoyl aspartate, disodium caproyl aspartate, potassium lauroyl aspartate, potassium myristoyl aspartate, potassium cocoyl aspartate, potassium caproyl aspartate, dipotassium lauroyl aspartate, dipotassium myristoyl aspartate, dipotassium cocoyl aspartate, and dipotassium caproyl aspartate and mixtures thereof.

8. Composition according to any of the preceding claims **characterised in that** at least one ethoxylated monoglyceride is selected from PEG-78 glyceryl cocoate, PEG-80 glyceryl cocoate, PEG-50 glyceryl isostearate, PEG-60 glyceryl isostearate, PEG-90 glyceryl isostearate, PEG-60 glyceryl stearate, PEG-120 glyceryl stearate, PEG-200 glyceryl stearate, PEG-80 glyceryl tallowate, PEG-82 glyceryl tallowate, PEG-130 glyceryl tallowate and PEG-200 glyceryl tallowate, and mixtures thereof.

9. Composition according to any of the preceding claims **characterised in that** it comprises at least one (poly)propylene glycol according to the following formula

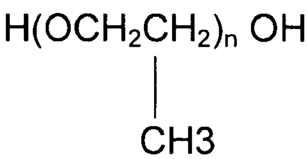

wherein n has a value between 1 and 70.

10. Composition according to any of the preceding claims **characterised in that** it comprises at least one conditioning agent.

11. Composition according to claim 10 **characterised in that** it comprises at least one cationic polymer as conditioning agent.

**12.** Composition according to claims 10 and 11 **characterised in that** it comprises oily substances as conditioning agent selected from silicone oils, either volatile or non-volatile, natural and synthetic oils.

**13.** Composition according to any of the preceding claims **characterised in that** it comprises at least one UV filter.

**14.** Composition according to any of the preceding claims **characterised in that** it comprises at least one direct dye.

**15.** Use of at least one ethoxylated monoglyceride according to the general formula of claim1 at a concentration of at least 1% by weight, in a cleansing composition based on at least one anionic surfactant, especially sulphate type of anionic surfactant, at a concentration of 2 to 25% by weight, and further comprising at least one amino acid surfactant according to above general formula of claim 1 at a concentration of 0.1 to 15% by weight, all values calculated to total composition, as shear thickening agent.

Europäisches Patentamt
European Patent Office
Office européen des brevets

# EUROPEAN SEARCH REPORT

Application Number
EP 08 00 7489

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br>Y | WO 03/013459 A (BEIERSDORF AG ET AL.)<br>20 February 2003 (2003-02-20)<br>* claims 1,4,5,7,8,12,13 *<br>* examples 6,9-12 *<br>* page 1, paragraph 1 *<br>* page 12, paragraph 3 *<br>* page 13, paragraph 3 *<br>----- | 1,2,4-8,<br>10-12,15<br><br><br><br><br><br>3 | INV.<br>A61K8/46<br>A61K8/44<br>A61K8/86<br>A61Q5/02<br>A61Q19/10 |
| X | WO 97/40125 A (UNILEVER PLC ET AL.)<br>30 October 1997 (1997-10-30)<br>* example 4; tables 3(C),3(D),2(B) *<br>* claims 1-3 *<br>* page 1, line 5 - line 17 *<br>* page 19, line 1 - page 20, line 3 *<br>* page 20, line 34 - page 21, line 13 *<br>* page 22, line 18 - line 35 *<br>----- | 1,4-9,15 | |
| Y | WO 2005/048971 A (BEIERSDORF AG ET AL.)<br>2 June 2005 (2005-06-02)<br>* example 22 *<br>* page 10, line 30 - page 11, line 5 *<br>* page 1, line 8 - line 10 *<br>* page 5, line 10 - page 6, line 6 *<br>----- | 3 | TECHNICAL FIELDS SEARCHED (IPC)<br>A61K<br>A61Q |
| D,A | WO 03/013467 A (BEIERSDORF AG ET AL.)<br>20 February 2003 (2003-02-20)<br>* page 1, paragraph 1 *<br>* page 3, paragraph 3 *<br>* page 4, paragraph 3 *<br>* page 7, paragraph 3 *<br>* examples 1-10 *<br>----- | 1-14 | |
| A | WO 2006/065878 A (ALZO INTERNATIONAL INC. ET AL.) 22 June 2006 (2006-06-22)<br>* page 1, line 6 - line 15 *<br>* page 21, line 4 - line 18 *<br>* examples 1-15 *<br>-----<br>-/-- | 15 | |

1

The present search report has been drawn up for all claims

| Place of search | Date of completion of the search | Examiner |
|---|---|---|
| Berlin | 7 October 2008 | Alvarez Alvarez, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 08 00 7489

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | WO 2004/024110 A (BEIERSDORF AG ET AL.) 25 March 2004 (2004-03-25)<br>* page 1, line 1 - line 17 *<br>* page 7, line 1 - line 8 *<br>* examples 1-6 *<br>----- | 1-14 | |
| D,A | WO 03/063818 A (BEIERSDORF AG ET AL.) 7 August 2003 (2003-08-07)<br>* page 1, line 1 - line 12 *<br>* page 5, line 32 - line 33 *<br>* page 8, line 13 - line 28 *<br>* page 9, line 11 - line 14 *<br>* page 9, line 25 - line 27 *<br>* examples 1-3 *<br>----- | 1-14 | |
| | | | TECHNICAL FIELDS SEARCHED (IPC) |

The present search report has been drawn up for all claims

1

| Place of search | Date of completion of the search | Examiner |
|---|---|---|
| Berlin | 7 October 2008 | Alvarez Alvarez, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 08 00 7489

This annex lists the patent family members relating to the patent documents cited in the above-mentioned European search report.
The members are as contained in the European Patent Office EDP file on
The European Patent Office is in no way liable for these particulars which are merely given for the purpose of information.

07-10-2008

| Patent document cited in search report | Publication date | Patent family member(s) | Publication date |
|---|---|---|---|
| WO 03013459 A | 20-02-2003 | EP 1420756 A2 | 26-05-2004 |
| | | JP 2005500361 T | 06-01-2005 |
| | | US 2004202635 A1 | 14-10-2004 |
| WO 9740125 A | 30-10-1997 | AU 725259 B2 | 12-10-2000 |
| | | AU 2024997 A | 12-11-1997 |
| | | BR 9708729 A | 03-08-1999 |
| | | CA 2248150 A1 | 30-10-1997 |
| | | DE 69713324 D1 | 18-07-2002 |
| | | DE 69713324 T2 | 03-04-2003 |
| | | EP 0900261 A1 | 10-03-1999 |
| | | US 5792739 A | 11-08-1998 |
| WO 2005048971 A | 02-06-2005 | DE 10354115 A1 | 21-07-2005 |
| | | EP 1686956 A1 | 09-08-2006 |
| | | JP 2007511579 T | 10-05-2007 |
| | | KR 20060121210 A | 28-11-2006 |
| | | US 2007292383 A1 | 20-12-2007 |
| WO 03013467 A | 20-02-2003 | EP 1418886 A1 | 19-05-2004 |
| | | JP 2005501065 T | 13-01-2005 |
| | | US 2004223939 A1 | 11-11-2004 |
| WO 2006065878 A | 22-06-2006 | CA 2590064 A1 | 22-06-2006 |
| | | EP 1830789 A2 | 12-09-2007 |
| WO 2004024110 A | 25-03-2004 | DE 10239712 A1 | 11-03-2004 |
| WO 03063818 A | 07-08-2003 | DE 10204099 A1 | 14-08-2003 |
| | | EP 1474102 A1 | 10-11-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03063818 A1
- WO 2004024110 A1
- WO 03013467 A1
- EP 70074 A
- EP 358216 A
- DE 2521960
- DE 2811010
- DE 3044738
- DE 3217059
- EP 337354 A
- EP 524612 A
- EP 640643 A
- US 3927199 A
- WO 2005065632 A1


### Non-patent literature cited in the description


- Hagers Handbuch der pharmazeutischen Praxis
- **Menzer, T.G.** Das Rheologie Handbuch. Vincentz Network GmbH, 2006, 246-250